# NEUE EUROPÄISCHE PATENTSCHRIFT

(11) **EP 2 257 363 B2**
(45) Veröffentlichungstag und Bekanntmachung des Hinweises auf die Entscheidung über den Einspruch: **27.02.2019**
(45) Hinweis auf die Patenterteilung: 16.05.2012
(21) Anmeldenummer: 09723090.8
(22) Anmeldetag: 13.03.2009
(51) Int. Cl.: B01D 61/18, A61M 1/10, A61M 1/36, A61M 39/22

(54) **VORSTERILISIERTES FILTRATIONSSYSTEM ZUM EINMALGEBRAUCH**
PRESTERILIZED FILTRATION SYSTEM FOR SINGLE USE
SYSTÈME DE FILTRATION PRÉSTÉRILISÉ POUR UTILISATION UNIQUE

(30) Priorität: 20.03.2008 DE 102008015387
(43) Veröffentlichungstag der Anmeldung: 08.12.2010
(73) Patentinhaber: Sartorius Stedim Biotech GmbH, 37079 Göttingen (DE)
(72) Erfinder: BÖTTCHER, Lars, 34212 Melsungen (DE); GAY, Isabelle, F-13124 Peypin (FR); GUTHOF, Frank, 34327 Körle (DE); SCHÄFER, Jan, 34295 Edermünde (DE)
(74) Vertreter: Schneider, Peter Christian
(86) Internationale Anmeldenummer: PCT/EP2009/001829
(87) Internationale Veröffentlichungsnummer: WO 2009/115242

(56) Entgegenhaltungen:
- WO-A-97/40867
- WO-A2-2006/122268
- US-A- 4 865 581
- US-A- 5 186 431
- US-A1- 2005 109 795
- US-A1- 2007 131 615
- US-B2- 6 712 963
- US-B2- 7 052 603

## Beschreibung

Die Erfindung betrifft ein vorsterilisierbares Filtrationssystem zum Einmalgebrauch, mit mindestens einem Behälter und mindestens einem Filtrationsmodul, die über ein durch mindestens ein Ventil regulierbares Schlauchsystem miteinander verbunden sind.

Aus der WO 97/40867, der US 6,712,963 B2 und aus der US 7,052,603 B2 sind Filtrationsanlagen bekannt, die vorsterilisierbare Filtrationssysteme zum Einmalgebrauch aufweisen. Die Filtrationssysteme bestehen aus einem mit einem Filtrationsmodul verbundenen Schlauchsystem, dass mit einem Rezirkulationsbehälter über aseptische Anschlüsse verbunden wird. Das Schlauchsystem bzw. das in dem Schlauchsystem von dem Filtrationsmodul zu dem Rezirkulationsbehälter transportierte Medium lässt sich über ein Klemmventil regulieren.

Bei einem solchen Klemmventil wird zur Absperrung des Schlauchs, der Schlauch von außen zusammengedrückt. Dies hat zwar einerseits den Vorteil, dass das Medium nicht mit dem Ventil in Kontakt kommt, andererseits hat es aber den Nachteil, dass insbesondere in der Produktion bei Verwendung von größeren und dickeren Schläuchen ein hoher Kraftaufwand des Ventils bzw. der auf den Schlauch wirkenden Klemmbakken notwendig ist. Das Schließverhalten beim Zusammenquetschen des Schlauchs kann zudem eine automatische Ventilregelung erschweren.

Die US 4 865 581 sowie die US 5 186 431 offenbaren Fluid systeme mit single-use Sensoren oder Pumpen.

Aufgabe der vorliegenden Erfindung ist es, das bekannte vorsterilisierbare Filtrationssystem zum Einmalgebrauch so zu verbessern, dass es zum einem gebrauchsfertig verpackt vorsterilisiert werden kann und um anderen sowohl im Labormaßstab als auch im Produktionsmaßstab in einer automatisierten Filtrationsanlage verwendet werden kann.

Diese Aufgabe wird, durch die Merkmals Kombination von Anspruch 1 gelöst.

Dadurch, dass das Ventil in einem separaten Ventilteil zum Einmalgebrauch und in einem wiederverwendbaren Stellantrieb aufgeteilt ist, wie auch in der WO 97/40867 offenbart, kann das separate Ventilteil unabhängig von der Wandstärke des Schlauchs in Hinblick auf seine Schließfunktion optimiert werden. Das zweigeteilte Ventil kann sowohl im Labormaßstab als auch im Produktionsmaßstab problemlos eingesetzt und über den Stellantrieb in Verbindung mit entsprechenden Sensoren geregelt werden. Das Ventilteil kann dabei als ein Klappenventil, ein Schieberventil oder beispielsweise ein Membranventil ausgebildet sein.

Gemäß der Erfindung ist der mindestens eine Behälter ein Rezirkulationsbehälter, der mit dem mindestens einen Filtrationsmodul und dem Schlauchsystem einen Kreislauf bildet.

Gemäß der Erfindung ist zwischen dem Rezirkulationsbehälter und dem Filtrationsmodul eine Pumpe angeordnet, die aus einem in das Schlauchsystem integrierten Pumpenteil zum Einmalgebrauch und aus einem wiederverwendbaren Pumpenantrieb ausgebildet ist.

Auch bei den bekannten Schlauchpumpen besteht das Problem, dass sie im größeren Maßstab, insbesondere bei Verwendung von relativ dicken Schläuchen, praktisch nicht eingesetzt werden können. In der Produktion würde dies unter Umständen zu einem Einsatz von Pumpen eines anderen Typs und zu einem Durchbrechen des Prinzips der Vorsterilisierung im geschlossen Kreislauf führen. Durch die Aufteilung der Pumpe in ein in das Schlauchsystem integriertes Pumpenteil zum Einmalgebrauch und in einen wieder verwendbaren Pumpenantrieb, kann relativ kostengünstig in unterschiedlichen Maßstäben der gleiche Pumpentyp unter Beibehaltung des Prinzips der Vorsterilisation eingesetzt werden.

Dabei kann das Pumpenteil als ein auswechselbarer Pumpenkopf einer Kreiselpumpe oder einer Druckkolben-/Kreiskolbenpumpe, wie sie z. B. aus der GB 2 440 944 A bekannt ist, ausgebildet sein.

Es ist aber beispielsweise auch möglich, das Pumpenteil als auswechselbaren Pumpenkopf einer Membranpumpe auszubilden, wie sie aus der DE 20 2006 020 237.4 bekannt geworden ist.

Nach einer bevorzugten Ausführungsform der 30 Erfindung ist der Rezirkulationsbehälter als ein flexibler Beutel ausgebildet. Der Rezirkulationsbehälter bzw. der flexible Beutel kann fest mit dem Schlauchsystem verbunden sein. Dies hat den Vorteil, dass auf aseptische bzw. sterile Anschlüsse, jedenfalls für die Hauptverbindung zum Rezirkulationsbehälter verzichtet werden kann. Eine spätere, flüssigkeitsdichte Trennung des Rezirkulationsbehälters oder beispielsweise eines Permeatbehälters kann in an sich bekannter Weise durch ein Durchtrennen des Verbindungsschlauchs mit einem Schweißgerät erfolgen, wobei die beiden Schlauchenden durch Verschweißen verschlossen werden.

Der Rezirkulationsbehälter ist in einer bevorzugten Ausführungsform der Erfindung mit einem Be-/Entgasungsfilter mit Drucksensor mit wieder verwendbarem Messwertaufnehmer beziehungsweise mit einem Sicherheitsventil ausgestattet, der den Rezirkulationsbehälter vor mechanischer Zerstörung durch unzulässigem Überdruck oder vor einem unerwünschten Kollabieren schützt.

Nach einer weiteren bevorzugten Ausführungsform der Erfindung sind der Permeatausgang des mindestens einen Filtrationsmoduls und ein Permeatbehälter über eine Permeatschlauchleitung fest verbunden. In der Permeatschlauchleitung kann ein Ventil angeordnet sein, das aus einem separaten Ventilteil zum Einmalgebrauch und aus einem wieder verwendbaren Stellantrieb ausgebildet ist.

Gemäß der Erfindung sind in dem Schlauchsystem und/oder der Permeatschlauchleitung Messstellen angeordnet, die jeweils in eine zum Einmalgebrauch in das Schlauchsystem und/oder der Permeatschlauchleitung integrierte Messzelle und einen wiederverwendbaren Messwertaufnehmer unterteilt sind.

Die produkt- bzw. medienberührten Teile der Messstellen können damit problemlos vorsterilisiert und mit dem gesamten Schlauchsystem entsorgt werden. Die Messwertaufnehmer bzw. Transmitter verbleiben in der Filtrationsanlage. Die Messzellen können dabei als Sensoren zur Druck-, Durchfluss-, Temperatur-, Füllstands-, pH-, Leitfähigkeits-, optischer Dichte-, Sauerstoffpartialdruck- und UV-Messung ausgebildet sein und ihre Signale über die Messwertaufnehmer an eine Rechen- und Kontrolleinheit weiterleiten.

Nach einer weiteren bevorzugten Ausführungsform der Erfindung ist das Filtrationsmodul als ein Dead-End-Filtrationsmodul ausgebildet. Als Filtrationsmodule kommen dafür insbesondere Filtercapsulen mit Hohlfasermembranen, Flachmembranen, Membranadsorbern und Tiefenfiltern für den Bereich der Ultrafiltration und Mikrofiltration in Betracht, einschließlich Virenfilter.

Nach einer weiteren bevorzugten Ausführungsform der Erfindung ist das Filtrationsmodul als ein tangential angeströmter Cross-Flow-Filter ausgebildet. Der Cross-Flow-Filter ist beispielsweise als ein Membrankassettenstapel ausgebildet. Als Filtrationsmodule kommen dafür insbesondere Membrankassetten mit Hohlfasermembranen, Flachmembranen, Membranadsorbern und Tiefenfiltern für den Bereich der Ultrafiltration und Mikrofiltration in Betracht, einschließlich Virenfilter.

Die Filtrationsmodule sind für den Einmalgebrauch, also als Wegwerfartikel ausgelegt.

Über aseptische Standardanschlüsse am Rezirkulationsbehälter können mit Hilfe von externen Aggregaten weitere Medien dem System zugeführt werden. Das vorsterilisierbare Filtrationssystem wird dem Betreiber sterilisiert und in steriler Umgebung verpackt geliefert. Das Filtrationssystem kann dabei im Ganzen aseptisch in die Filtrationsanlage eingebaut und betrieben werden. Damit kann eine Filtration aseptisch bzw. steril mit Einwegprodukten betrieben werden, wobei nach erfolgter Filtration alle produktberührten Bauteile als geschlossenes System entsorgt werden können. Ein Austreten von Medien aus dem System ist damit zum Schutz des Betreibers ausgeschlossen. Die restliche Filtrationsanlage mit der Datenerfassung und Visualisierung, beispielsweise über einen Bildschirm, welcher vorzugsweise als ein Berührungsbildschirm (Touch-Screen) ausgebildet ist, sowie die mechanischen Versorgungseinheiten werden bei nachfolgenden Produktionszyklen mit einem neuen sterilen Filtrationssystem bestückt.

Alle Messstellen bzw. Messzellen können dem Betreiber vorkalibriert im Rahmen des Filtrationssystems zur Verfügung gestellt werden.

Das vorsterilisierbares Filtrationssystem ist als geschlossenes System steril verpackt. Sämtliche Komponenten des an den Kunden lieferbaren Filtrationssystems sind sterilisierbar, wobei die Sterilisierung vorzugsweise durch Gammastrahlen erfolgt ist.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:

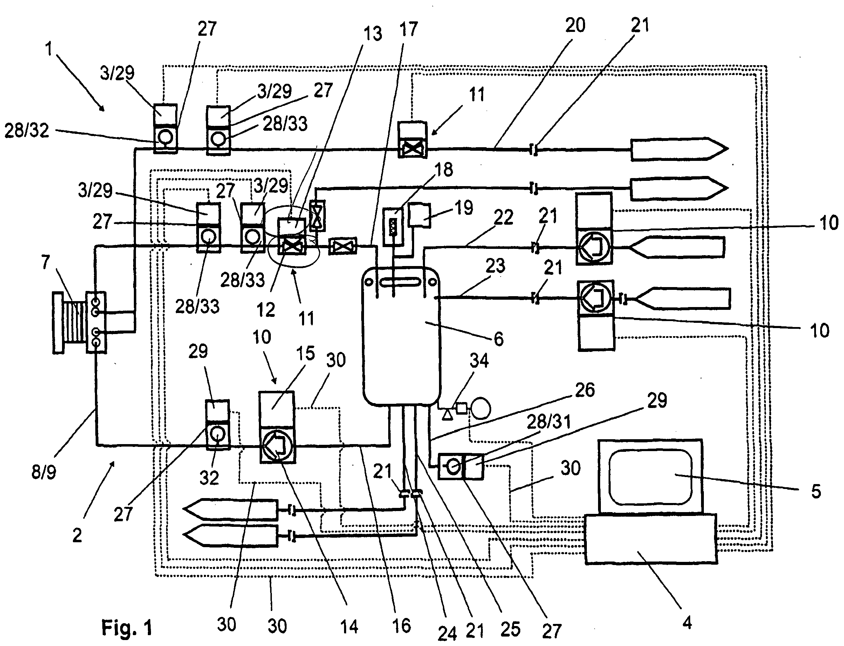

Figur 1: eine schematische Darstellung einer Filtrationsanlage und

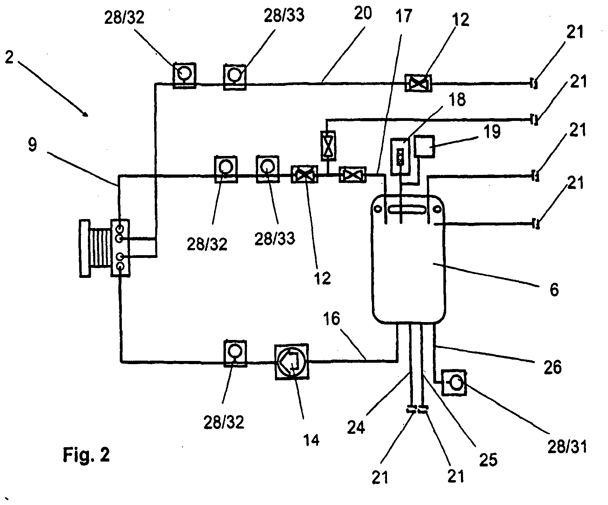

Figur 2: eine schematische Darstellung des vorsterilisierbaren Filtrationssystems von Figur 1.

Die Filtrationsanlage 1 besteht im Wesentlichen aus einem vorsterilisierbaren Filtrationssystem 2 zum Einmalgebrauch und wiederverwendbaren Anlageteilen 3 einschließlich einer Rechen- und Kontrolleinheit 4 mit einem Bildschirm 5 zur Visualisierung.

Das Filtrationssystem 2 ist zum Einmalgebrauch ausgebildet und besteht im Wesentlichen aus einem Rezirkulationsbehälter 6 und einem Filtrationsmodul 7, die über ein Schlauchsystem 8 in einem Kreislauf 9 miteinander verbunden sind. Zwischen dem Rezirkulationsbehälter 6 und dem Filtrationsmodul 7 ist zur Zirkulation des zu filtrierenden Mediums eine Pumpe 10 angeordnet. Zwischen dem Filtrationsmodul 7 und dem Rezirkulationsbehälter 6 ist im Schlauchsystem 8 bzw. Kreislauf 9 ein von der Rechen- und Kontrolleinheit 4 steuerbares Ventil 11 angeordnet. Das Ventil 11 besteht aus einem separaten Ventilteil 12, mit einem veränderbaren Durchlass, durch den das Medium hindurchgeleitet wird, und aus einem mit dem Ventilteil 12 verbindbaren Stellantrieb 13, der ein wiederverwendbares Anlagenteil 3 bildet. Der Stellantrieb 13 ist mit der Rechen- und Kontrolleinheit 4 verbunden und wird von dieser gesteuert. Das Ventilteil 12 ist beispielsweise als ein nicht weiter dargestelltes Klappenventil ausgebildet.

Die Pumpe 10 ist ebenfalls zweiteilig ausgebildet und besteht aus einem in das Schlauchsystem 8 integrierten Pumpenteil 14 zum Einmalgebrauch und aus einem wiederverwendbaren Pumpenantrieb 15, der ein wiederverwendbares Anlagenteil bildet und der ebenfalls mit der Rechen- und Kontrolleinheit 4 verbunden ist und von dieser gesteuert wird. Das Pumpenteil 14 ist beispielsweise als ein nicht weiter dargestellter Pumpenkopf einer Kreiselpumpe ausgebildet.

Der Rezirkulationsbehälter 6 ist als ein flexibler Beutel ausgebildet und fest mit einem zum Pumpenteil 14 hinführenden Vorlaufschlauch 16 und mit einem vom Filtrationsmodul 7 rückführenden Rücklaufschlauch 17 des Kreislaufs 9 fest verbunden.

Das Filtrationsmodul 7 ist mit dem Kreislauf 9 verbunden. Die Permeatauslässe des Filtrationsmoduls 7 sind mit einer Permeatschlauchleitung 20 verbunden, die zu einem nicht weiter dargestellten Permeatbehälter führt. In die Permeatschlauchleitung 20 ist ein separates Ventilteil 12 und/oder ein weiteres regelbares Ventil 11 integriert. In der Permeatschlauchleitung 20 ist zum nicht dargestellten Permeatbehälter hin ein Sterilverbinder 21 angeordnet, über den der Permeatbehälter steril angekoppelt bzw. steril oder zumindest aseptisch ausgetauscht werden kann. Vorzugsweise ist der Permeatbehälter fest mit der Permeatschlauchleitung 20 verbunden. Der Rezirkulationsbehälter 6 weist an seinem oberen Ende zwei Schlauchanschlüsse 22, 23 auf, die an ihren Enden von Sterilverbindern 21 verschlossen werden und über die ggf. mit Hilfe von weiteren Pumpen 10 Medien zugeführt werden können. An seinem unteren Ende weist der Rezirkulationsbehälter 6 zwei weitere Schlauchanschlüsse 24, 25 auf, die ebenfalls durch Sterilverbinder 21 abgeschlossen werden und an die jeweils nicht dargestellte Behälter angeschlossen werden können um Medium aus dem Rezirkulationsbehälter 6 aufzunehmen. Ein weiterer an dem unteren Ende des Rezirkulationsbehälters 6 angeordneter Schlauch 26 ist mit einer Messstelle 27 verbunden, die in eine zum Einmalgebrauch in das Schlauchsystem 8 integrierte Messzelle 28 und einen wiederverwendbaren Messwertaufnehmer 29 unterteilt ist. Der Messwertaufnehmer 29 ist jeweils über eine Signalleitung 30 mit der Rechen- und Kontrolleinheit 4 verbunden.

Der Rezirkulationsbehälter 6 ist mit einem Be-/Entgasungsfilter 18 mit einem Sicherheitsventil 19 ausgestattet.

Die in den Schlauch 26 integrierte Messzelle 28 ist als ein Temperatursensor 31 ausgebildet. Im Kreislauf 9 ist zwischen Pumpe 10 und Filtrationsmodul 7 eine weitere Messstelle 27 angeordnet, deren Messzelle 28 als ein Drucksensor 32 ausgebildet ist. Weiterhin sind in dem Kreislauf 9 zwischen dem regelbaren Ventil 11 und dem Filtrationsmodul 7 zwei weitere Messstellen 27 angeordnet. Dabei ist eine Messzelle 28 als ein Drucksensor 32 und eine weitere Messzelle 28 als ein Flussmesser 33 ausgebildet.

In der Permeatschlauchleitung 20 sind ebenfalls zwei Messstellen 27 angeordnet, wobei die Messzelle 28 der einen als Drucksensor 32 und die Messzelle 28 der anderen Messstelle 27 als Flussmesser 33 ausgebildet ist. Zum Sterilverbinder 21 hin ist in der Permeatschlauchleitung 20 ein weiteres regelbares Ventil 11 angeordnet. Der Rezirkulationsbehälter 6 ist mit einem Wägemodul 34 verbunden, dass das Gewicht im Rezirkulationsbehälter 6 ermittelt und an die Rechen- und Kontrolleinheit 4 weiterleitet. Das Signal des Wägemoduls 34 kann auch als Füllstandssignal genutzt werden.

Das Filtrationssystem 2 wird als komplett miteinander verbundenes Gesamtsystem vorsterilisiert und in steriler Umverpackung geliefert. Der Betreiber muss dann lediglich das Filtrationssystem 2 aus seiner nicht dargestellten Umverpackung entnehmen und in die Filtrationsanlage 1 einsetzen und die Einwegteile der Ventile 11, Pumpen 10 und Messstellen 27 mit ihren in der Filtrationsanlage 1 verbliebenen Gegenstücken verbinden.

Nach erfolgter Filtration können alle produktberührten Bauteile des Filtrationssystems 2 als geschlossenes System wieder entsorgt werden.

Alle Messzellen 28 der Messstellen 27 werden dem Betreiber vorkalibriert zur Verfügung gestellt.

## Patentansprüche

1. Vorsterilisierbares Filtrationssystem (2) zum Einmalgebrauch, mit mindestens einem Behälter (6) und mindestens einem Filtrationsmodul (7), die über ein durch mindestens ein Ventil (11) regulierbares Schlauchsystem (8) miteinander verbunden sind,
**dadurch gekennzeichnet,**
**dass** das regelbare Ventil (11) aus einem in das Schlauchsystem (8) integrierten, vom Schlauchsystem (8) separaten Ventilteil (12) zum Einmalgebrauch mit einem veränderbaren Durchlass und aus einem wieder verwendbaren Stellantrieb (13) ausgebildet ist,
**dass** in dem Schlauchsystem (8) Messstellen (27) angeordnet sind, die jeweils in eine zum Einmalgebrauch in das Schlauchsystem integrierte Messzelle (28) und einen wieder verwendbaren Messwertaufnehmer (29) unterteilt sind,
**dass** der mindestens eine Behälter (6) ein Rezirkulationsbehälter ist, der mit dem mindestens einen Filtrationsmodul (7) und dem Schlauchsystem (8) einen Kreislauf (9) bildet,
**dass** zwischen dem mindestens einen Rezirkulationsbehälter und dem mindestens einen Filtrationsmodul (7) eine Pumpe (10) angeordnet ist, die aus einem in das Schlauchsystem (8) integrierten Pumpenteil (14) zum Einmalgebrauch und aus einem wieder verwendbaren Pumpenantrieb (15) ausgebildet ist,
**dass** das Pumpenteil (14) als auswechselbarer Pumpenkopf einer Druckkolben-pumpe, Kreiskolbenpumpe, Kreiselpumpe oder Membranpumpe ausgebildet ist, und
**dass** das Filtrationssystem als geschlossenes System steril verpackt ist und im Ganzen aseptisch in eine Filtrationsanlage einbaubar ist.

2. Vorsterilisierbares Filtrationssystem nach Anspruch 1,
**dadurch gekennzeichnet,**
**dass** das Ventilteil (12) als ein Klappenventil, Membranventil, Kugelhahnventil, Sitzventil oder Schieberventil ausgebildet ist.

3. Vorsterilisierbares Filtrationssystem nach Anspruch 1 oder 2,
**dadurch gekennzeichnet,**
**dass** der mindestens eine Rezirkulationsbehälter als ein flexibler Beutel ausgebildet ist.

4. Vorsterilisierbares Filtrationssystem nach einem der Ansprüche 1 bis 3,
**dadurch gekennzeichnet,**
**dass** der mindestens eine Rezirkulationsbehälter über eine feste Verbindung mit dem Schlauchsystem (8) verbunden ist und dass der mindestens eine Rezirkulationsbehälter mit einem Schweißgerät flüssigkeitsdicht von dem Schlauchsystem (8) trennbar ist.

5. Vorsterilisierbares Filtrationssystem nach einem der Ansprüche 1 bis 4,
**dadurch gekennzeichnet,**
**dass** der Permeatausgang des mindestens einen Filtrationsmoduls (7) und ein Permeatbehälter über eine Permeatschlauchleitung (20) fest verbunden sind.

6. Vorsterilisierbares Filtrationssystem nach Anspruch 5,
**dadurch gekennzeichnet,**
**dass** in der Permeatschlauchleitung (20) ein Ventil (11) aus einem separaten Ventilteil (12) zum Einmalgebrauch und aus einem wieder verwendbaren Stellantrieb (13) ausgebildet ist.

7. Vorsterilisierbares Filtrationssystem nach Anspruch 1,
**dadurch gekennzeichnet,**
**dass** das mindestens eine Filtrationsmodul (7) als Dead-End-Filtrationsmodul ausgebildet ist.

8. Vorsterilisierbares Filtrationssystem nach Anspruch 7,
**dadurch gekennzeichnet,**
**dass** das Dead-End-Filtrationsmodul (7) als eine Filtercapsule ausgebildet ist.

9. Vorsterilisierbares Filtrationssystem nach Anspruch 1,
**dadurch gekennzeichnet,**
**dass** das mindestens eine Filtrationsmodul (7) als ein tangential angeströmter Crossflow-Filter ausgebildet ist.

10. Vorsterilisierbares Filtrationssystem nach Anspruch 9,
**dadurch gekennzeichnet,**
**dass** der Crossflow-Filter (7) als Membrankassettenstapel ausgebildet ist.

11. Vorsterilisierbares Filtrationssystem nach einem der Ansprüche 1 bis 10,
**dadurch gekennzeichnet,**
**dass** das mindestens eine Filtrationsmodul (7) Filtrationsmembranen im Bereich der Ultrafiltration oder Mikrofiltration in Form von Hohlfasern, Flachmembranen oder Membranadsorbern enthält.

12. Vorsterilisierbares Filtrationssystem nach Anspruch 1,
**dadurch gekennzeichnet,**
**dass** der mindestens eine Rezirkulationsbehälter (6) mit einem Sicherheitsventil für den Einmalgebrauch ausgestattet ist.

13. Vorsterilisierbares Filtrationssystem nach einem der Ansprüche 1 bis 12,
**dadurch gekennzeichnet,**
**dass** das gesamte Filtrationssystem mit Gammastrahlen sterilisierbar ist.

## Claims

1. A presterilizable filtration system (2) for single use having at least one container (6) and at least one filtration module (7) that are connected with each other via a hose system (8) that is regulated by at least one valve (11), **characterized in that**
the regulatable valve (11) is formed by a single-use valve part (12) that is integrated into the hose system (8), said valve part being separate from the hose system (8) and having a variable passage, and by a reusable actuator (13), further **characterized in that**
measuring points (27) are arranged in the hose system (8), each of which being subdivided into a single-use measuring cell (28) which is integrated into the hose system, and into a reusable transducer (29),
further **characterized in that**
the at least one container (6) is a recirculation tank that forms a circuit (9) together with the at least one filtration module (7) and the hose system (8), further **characterized in that**
a pump (10) is arranged between the at least one recirculation tank and the at least one filtration module, said pump consisting of a single-use pump part (14) that is integrated into the hose system (8) and also of a reusable pump drive (15), further **characterized in that**
the pump part (14) is configured as the exchangeable pump head of a pressure piston pump, rotary piston pump, centrifugal pump, or diaphragm pump,
and
further **characterized in that**
the filtration system can be packaged in a sterile manner as a closed system and the whole can be aseptically incorporated into a filtration plant.

2. A presterilizable filtration system according to Claim 1,
**characterized in that**
the valve part (12) is configured as a flap valve, diaphragm valve, ball valve, seat valve, or slide valve.

3. A presterilizable filtration system according to Claim 1 or 2,
**characterized in that**
the at least one recirculation tank is configured as a flexible bag.

4. A presterilizable filtration system according to any of Claims 1 to 3,
**characterized in that**
the at least one recirculation tank is connected with the hose system (8) via a fixed connection, and
further **characterized in that**
the at least one recirculation tank can be separated in a liquid-tight manner from the hose system (8) by using a welding appliance.

5. A presterilizable filtration system according to any of Claims 1 to 4.
**characterized in that**
the permeate outlet of the at least one filtration module (7) and a permeate container are fixedly connected to each other via a permeate hose line (20).

6. A presterilizable system according to Claim 5,
**characterized in that**
in the permeate hose line (20) a valve (11) is made up of a separate single-use valve part (12) and a reusable actuator (13).

7. A presterilizable filtration system according to Claim 1,
**characterized in that**
the at least one filtration module (7) is configured as a dead-end filtration module.

8. A presterilizable filtration system according to Claim 7,
**characterized in that**
the dead-end filtration module (7) is configured as a filter capsule.

9. A presterilizable filtration system according to Claim 1,
**characterized in that**
the at least one filtration module (7) is configured as a cross-flow filter with tangential feed.

10. A presterilizable filtration system according to Claim 9,
**characterized in that**
the cross-flow filter (7) is configured as a stack of membrane cartridges.

11. A presterilizable filtration system according to any of Claims 1 to 10,
**characterized in that**
the at least one filtration module (7) contains filtration membranes in the form of hollow fibres, flat membranes or membrane adsorbers in the ultrafiltration or microfiltration range.

12. A presterilizable filtration system according to Claim 1,
**characterized in that**
the at least one recirculation tank (6) is equipped with a safety valve for single use.

13. A presterilizable filtration system according to any of Claims 1 to 12, **characterized in that**
the entire filtration system can be sterilized by gamma rays.

## Revendications

1. Système de filtration préstérilisable (2) pour utilisation unique ayant au moins un contenant (6) et au moins un module de filtration (7) raccordés l'un à l'autre par un système de flexible (8) réglable par au moins une vanne (11),
**caractérisé en ce que** la vanne réglable (11) est constituée d'une partie vanne (12) pour utilisation unique intégrée au système de flexible (8), ladite partie vanne étant séparée du système de flexible (8) et ayant un passage variable, et d'un actionneur réutilisable (13),
**caractérisé en ce que** des points de mesure (27) sont disposés dans le système de flexible (8), chacun étant subdivisé en une cellule de mesure (28) pour utilisation unique intégrée au système de flexible et en un enregistreur de mesures (29) réutilisable,
**caractérisé en ce que** la cuve (6) au nombre minimum de 1 est une cuve de recirculation qui forme un circuit (9) conjointement avec le module de filtration (7) au nombre minimum de 1 et le système de flexible (8).
**caractérisé en ce qu'**une pompe (10) est disposée entre la cuve de recirculation au nombre minimum de 1 et le module de filtration (7) au nombre minimum de 1, ladite pompe étant constituée d'une partie pompe (14) pour utilisation unique intégrée au système de flexible (8) et d'un entraînement de pompe (15) réutilisable,
**caractérisé en ce que** la partie pompe (14) est configurée en tant que tête de pompe interchangeable d'une pompe à piston à pression, d'une pompe à piston rotatif, d'une pompe centrifuge ou d'une pompe à membrane, et
**caractérisé en ce que** le système de filtration est conditionné de façon stérile en tant que système clos et peut être incorporé aseptiquement en bloc dans une installation de filtration.

2. Système de filtration préstérilisable selon la revendication 1,
**caractérise en ce que** la partie vanne (12) est configurée en tant que vanne à clapet, vanne à membrane, vanne à boisseau sphérique, vanne à siège ou vanne à coulisse.

3. Système de filtration préstérilisable selon la revendication 1 ou 2,
**caractérisé en ce que** la cuve de recirculation au nombre minimum de 1 est configurée en tant que sac souple,

4. Système de filtration préstérilisable selon l'une quelconque des revendications 1 à 3,
**caractérisé en ce que** la cuve de recirculation au nombre minimum de 1 est raccordée au système de flexible (8) par un raccord fixe, et **en ce que** la cuve de recirculation au nombre minimum de 1 peut être séparé du système de flexible (8) de manière étanche aux liquides avec un dispositif de soudage.

5. Système de filtration préstérilisable selon l'une quelconque des revendications 1 à 4,
**caractérisé en ce que** la sortie de perméat du module de filtration (7) au nombre minimum de 1 et une cuve perméat sont fixées l'une à l'autre par une conduite de perméat (20).

6. Système de filtration préstérilisable selon la revendication 5,
**caractérisé en ce que**, dans la conduite de perméat (20), une vanne (11) est constituée d'une partie vanne (12) séparée pour utilisation unique et d'un actionneur (13) réutilisable.

7. Système de filtration présterilisable selon la revendication 1,
**caractérisé en ce que** le module de filtration (7) au nombre minimum de 1 est configuré en tant que module de filtration en cul-de-sac.

8. Système de filtration préstérilisable selon la revendication 7,
**caractérisé en ce que** le module de filtration en cul-de-sac (7) est configuré comme une capsule filtrante.

9. Système de filtration préstérilisable selon la revendication 1
**caractérisé en ce que** le module de filtration (7) au nombre minimum de 1 est configuré en tant que filtre transversal à courant tangentiel.

10. Système de filtration préstérilisable selon la revendication 9,
**caractérisé en ce que** le filtre transversal (7) est configuré en tant que pile de cartouches à membrane.

11. Système de filtration préstérilisable selon l'une quelconque des revendications 1 à 10,
**caractérisé en ce que** le module de filtration (7) au nombre minimum de 1 contient des membranes de filtration sous forme de fibres creuses, de membranes plates ou d'adsorbeurs à membrane au niveau de l'ultrafiltration ou de la microfiltration.

12. Système de filtration préstérilisable selon la revendication 1,
**caractérisé en ce que** la cuve de recirculation au nombre minimum de 1 est équipé d'une vanne de sûreté pour utilisation unique.

13. Système de filtration préstérilisable selon l'une quelconque des revendications 1 à 12,
**caractérisé en ce que** l'ensemble du système de filtration peut être stérilisé par des rayons gamma.
